(19) **Europäisches Patentamt / European Patent Office / Office européen des brevets**

(11) **EP 4 668 279 A1**

(12) **EUROPEAN PATENT APPLICATION**
published in accordance with Art. 153(4) EPC

(43) Date of publication:
**24.12.2025 Bulletin 2025/52**

(21) Application number: **23922712.7**

(22) Date of filing: **16.02.2023**

(51) International Patent Classification (IPC):
**G16B 40/00** (2019.01)

(52) Cooperative Patent Classification (CPC):
**G16B 40/00**

(86) International application number:
**PCT/JP2023/005471**

(87) International publication number:
**WO 2024/171375 (22.08.2024 Gazette 2024/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **FUJITSU LIMITED**
**Kawasaki-shi, Kanagawa 211-8588 (JP)**

(72) Inventor: **KURIBAYASHI, Sotaro**
**Kawasaki-shi, Kanagawa 211-8588 (JP)**

(74) Representative: **Hoffmann Eitle**
**Patent- und Rechtsanwälte PartmbB**
**Arabellastraße 30**
**81925 München (DE)**

(54) **INFORMATION PROCESSING PROGRAM, INFORMATION PROCESSING METHOD, AND INFORMATION PROCESSING DEVICE**

(57) An information processing program causes a computer to execute a process including: obtaining a third feature based on statistical information, the statistical information being obtained by prediction of each of amino acids included in a protein corresponding to input data including a first feature related to a three-dimensional structure of a protein of a virus and a second feature related to a property originated from the three-dimensional structure, the prediction being performed by inputting the input data into a machine-learning model (103), and training a regression model (110) that predicts an amino-acid sequence of the virus after mutation using the second feature and the third feature as an input feature.

FIG.1

**EP 4 668 279 A1**

**Description**

[Technical Field]

The present embodiment relates to an information processing program, an information processing method, and an information processing device.

[Background Art]

Since a virus frequently mutates, prediction of mutation is an important issue in developing vaccines against virus such as coronavirus.

Some conventional methods have predicted the amino-acid sequence after mutation by means of time-series analysis that regards a protein of a virus as an amino-acid sequence and associates the protein with the time of the epidemic, or LSTM (Long Short-Term Memory).

[Prior Art Reference]

[Patent Document]

[Patent Literature 1] International Publication Pamphlet No. 2022/019331
[Patent Literature 2] Japanese National Publication of International Patent Application No. 2022-521686
[Patent Literature 3] U.S. Patent Application Publication No. 2012/0265513
[Patent Literature 4] Japanese National Publication of International PatentApplication No. 2022-527381
[Patent Literature 5] U.S. Patent Application Publication No. 2019/0266493

However, such conventional methods for predicting virus mutation have difficulty in reflecting the influences between amino acids structurally distant from each other and the difference in the properties of the same amino acid located different positions in the virus when predicting the virus mutation.

Even if having the same chemical formula, some compounds, such as isomers, have different property and formation. Such conventional methods for predicting virus mutation have difficulty in following the compounds. Therefore, the accuracy in predicting virus mutation may be degraded.

According to an aspect, one of the objects of the present disclosure is to enhance the accuracy in predicting virus mutation.

[Means to Solve the Problem]

For the above, an information processing program causes a computer to execute a process including: obtaining a third feature based on statistical information, the statistical information being obtained by prediction of each of amino acids included in a protein corresponding to input data including a first feature related to a three-dimensional structure of a protein of a virus and a second feature related to a property originated from the three-dimensional structure, the prediction being performed by inputting the input data into a machine-learning model, and training a regression model that predicts an amino-acid sequence of the virus after mutation using the second feature and the third feature as an input feature.

[Effect of Invention]

According to the embodiment, the accuracy in predicting mutation of a virus can be enhanced.

[Brief Description of Drawings]

FIG. 1 is a schematic diagram illustrating a configuration of an information processing device according to one embodiment;
FIG. 2 is a diagram illustrating an amino-acid sequence and an antigen cluster name used in the information processing device of the one embodiment;
FIG. 3 is a block diagram illustrating an example of a hardware configuration of a computer that achieves a function of the information processing device of the one embodiment;

FIG. 4 is a diagram showing amino-acid 3D structure information output from a 3D structure calculating processor in the information processing device of the one embodiment;

FIG. 5 is a diagram illustrating chemical parameter information that a chemical parameter calculating processor generates in the information processing device of the one embodiment;

FIG. 6 is a diagram illustrating graph information in the information processing device of the one embodiment;

FIG. 7 is a diagram illustrating a process that a graph data shaping processor carries out in the information processing device of the one embodiment;

FIG. 8 is a diagram illustrating graph AI input information in the information processing device of the one embodiment;

FIG. 9 is a diagram illustrating statistical information in the information processing device of the one embodiment;

FIG. 10 is a diagram illustrating a process that a graph AI calculating processor carries out in a predicting phase in the information processing device of the one embodiment;

FIG. 11 is a diagram illustrating a process that an amino-acid sequence calculating processor carries out in the information processing device of the one embodiment;

FIG. 12 is a flow chart illustrating a process performed in the information processing device of the one embodiment; and

FIG. 13 is a flow chart illustrating a process that the graph AI calculating processor carries out in the information processing device of the one embodiment;

[Description of Embodiments]

Hereinafter, description will now be made in relation to a program for processing information, a method for processing information, and an information processing device according to the present embodiment with reference to the accompanying drawings. However, the following embodiment is merely illustrative and is not intended to exclude the application of various modifications and techniques not explicitly described in the embodiment. Namely, the present embodiment can be variously modified and implemented without departing from the scope thereof. Further, each of the drawings can include additional functions not illustrated therein to the elements illustrated in the drawing.

Configuration:

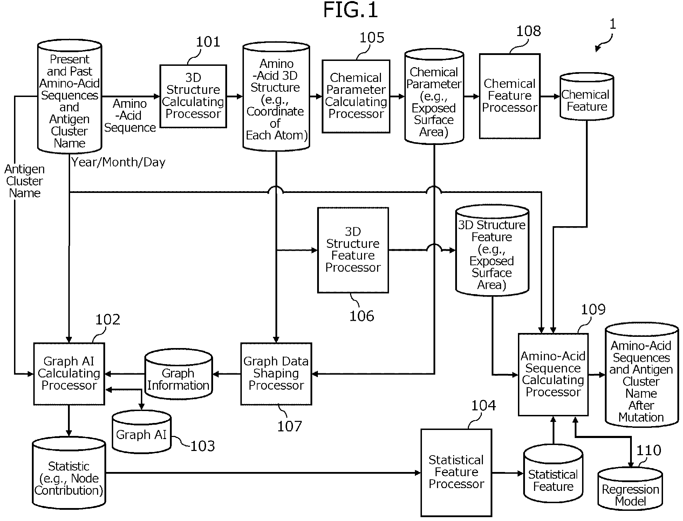

FIG. 1 is a diagram schematically illustrating a configuration of an information processing device 1 according to one embodiment.

The present information processing device 1 performs training (machine-learning) of a regression model (machine learning model) 110 that predicts an amino-acid sequence of a protein of a virus after mutation (training phase).

In the training phase, an amino-acid sequence and an antigen cluster name of a virus at a certain past time point are input into the information processing device 1, and the amino-acid sequence and the antigen cluster name of the virus (i.e., mutated virus) after the mutation are used as correct answer data.

Such a virus at a certain past time point may be simply referred to as a "past virus". In addition, an amino acid contained in this past virus may be referred to as a "past amino acid". An antigen cluster name may be simply referred to as a "cluster name". In addition, an amino-acid sequence and an antigen cluster of a past virus may be referred to as a "past amino-acid sequence" and a "past antigen cluster name".

In addition, the present information processing device 1 uses the trained regression model 110 for prediction (inference) of an amino-acid sequence of the protein of a mutated virus (predicting phase).

In predicting phase, an amino-acid sequence and an antigen cluster name of a current (latest) virus are input into the information processing device 1, and the regression model 110 predicts the amino-acid sequence and the antigen cluster name of the same virus (i.e., mutated virus) after the mutation. In the predicting phase, such an amino-acid sequence and an antigen cluster name of the same virus after the mutation that the regression model 110 predicts on the basis of the input amino-acid sequence and the input antigen cluster name of the current (latest) virus may be referred to as a future amino-acid sequence and a future antigen cluster name.

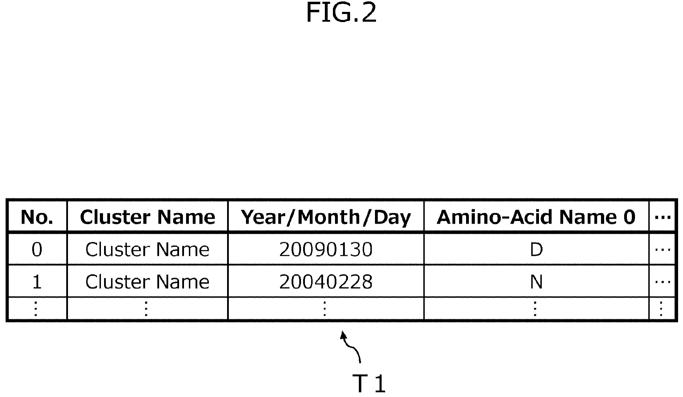

FIG. 2 is a diagram illustrating an amino-acid sequence and an antigen cluster name information used in the information processing device 1 according to the one embodiment.

In FIG. 2, the amino-acid sequence and antigen cluster name information is represented in a data table format. Hereinafter, the amino-acid sequence and antigen cluster name information is sometimes represented by attaching thereto the reference sign T1.

The amino-acid sequence and antigen cluster name information T1 illustrated in FIG. 2 associates No., cluster name, year/month/day, and an amino-acid names with one another.

In the amino-acid sequence and antigen cluster name information T1 illustrated in FIG. 2, each of the data pieces represented by a letter string for convenience may be practically an integer value uniquely associated with the data piece.

Data expressed in an integral value can be used efficiently for various computations and is highly convenient.

The field of "No." represents information for identifying a virus. The field of "cluster name" represents an antigen cluster name of the virus. The field of "year/month/day" may represent the date and time when the virus appeared or was discovered. The amino-acid name indicates the type of amino acid contained in the virus, and represents any one of the 20 types of amino acids. In FIG. 2, for convenience, the amino-acid names (amino acid types) are represented using the letters, such as D and N.

If a virus includes multiple amino acids, the amino-acid sequence and antigen cluster name information T1 may list the multiple amino-acid names in association with the virus. The order of the amino-acid names may be, for example, arranged from the beginning to the end in the order of the peptide bond.

The multiple amino acids contained in a virus may be represented by numbers. A number representing an amino acid contained in a virus may be referred to as an amino-acid number. In the example illustrated in FIG. 2, the amino-acid number 0, which attaches an amino-acid number "0" to an amino-acid name, represents the 0-th amino acid among multiple amino acids included in a virus.

The amino-acid sequence and antigen cluster name information T1 may be prepared by a user, for example. In addition, for example, a non-illustrated processor may generate the amino-acid sequence and antigen cluster name information T1 by extracting information of an amino acid and an antigenic cluster name from information of a known virus.

Example of Hardware Configuration:

The function of the information processing device 1 of the one embodiment may be achieved by one computer or by two or more computers. Further, at least a part of the functions of the information processing device 1 may be implemented using Hardware (HW) resources and Network (NW) resources provided by cloud environment.

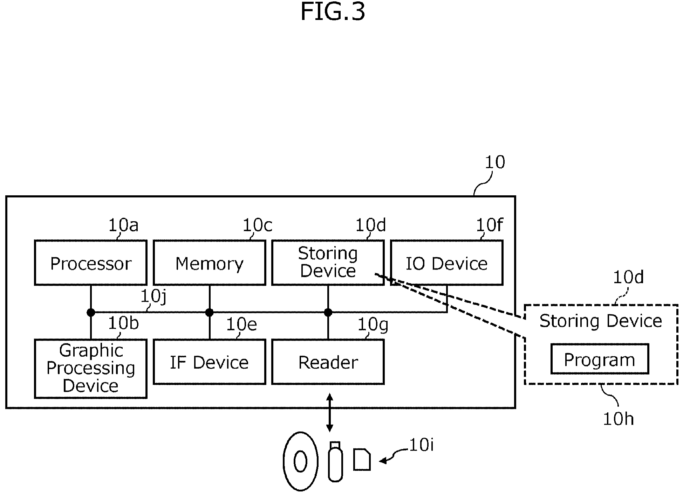

FIG. 3 is a block diagram illustrating an example of a hardware (HW) configuration of the computer 10 that achieves the function of information processing device 1 according to the one embodiment. If multiple computers are used as the HW resources for achieving the functions of the information processing device 1, each of the computers may include the HW configuration illustrated in FIG. 3.

As illustrated in FIG. 3, the computer10 may illustratively include, as the HW configuration, a processor 10a, a graphic processing device 100b, a memory 10c, a storing device 10d, an Interface (IF) device 10e, an Input/Output (IO) device 10f, and a reader 10g.

The processor 10a is an example of an arithmetic processing device that performs various types of control and calculations and serving as a controller that carries out various processes. The processor 10a may be mutually communicably connected to each of the blocks in the computer 10 via a bus 10j. The processor 10a may be a multiprocessor including multiple processors or a multi-core processor including multiple processor cores, or may have a structure including two or more multi-core processors.

The processor 10a may be any one of integrated circuits (ICs) such as CPUs (Central Processing Units), MPUs (Micro Processing Units), APUs (Accelerated Processing Units), DSPs (Digital Signal Processors), ASICs (Application Specific Integrated Circuits), and FPGAs (Field Programmable Gate Arrays), or combinations of two or more of these ICs.

The graphic processing device 10b carries out screen displaying control on an output device such as a monitor serving as one of the IO device 10f. The graphic processing device 10b may have a function as an accelerator that executes a machine learning process and a predicting process using a machine learning model. Examples of the graphic processing device 10b are various ICs such as Graphic Processing Units (GPUs), APUs, DSPs, ASICs and FGPAs.

The memory 10c is an example of a hardware device that stores various pieces of data and information of a program. Examples of the memory 10c are one of a volatile memory such as a Dynamic Random Access Memory (DRAM) and a non-volatile memory such as a persistent Memory (PM) or the both.

The storing device 10d is an example of a hardware device that stores information such as various data, programs, and the like. Examples of the storing device 10d may be various storing devices including a magnetic disk device such as a Hard Disk Drive (HDD), a semiconductor drive device such as a Solid State Drive (SSD), a nonvolatile memory, and the like. The non-volatile memory may be, for example, a flash memory, a Storage Class Memory (SCM), a Read Only Memory (ROM), and the like.

The storing device 10d may store a program 10h (information processing program) that implements all or a part of various functions of the computer 10.

For example, the processor 10a of the information processing device 1 may achieve the function in a training phase and the function in a predicting phase to be detailed below by expanding the program 10h stored in the storing device 10d onto the memory 10c and executing the expanded program 10h.

The IF device 10e is an example of a communication IF that controls connections and communications between the computer 10 and other computer. For example, the IF device 10e may include an applying adapter conforming to Local Area Network (LAN) such as Ethernet® or optical communication such as Fibre Channel (FC). The applying adapter may be compatible with either or both of wireless and wired communication schemes.

For example, the computer 10 may be communicably connected to a non-illustrated another computer and a database via the IF device 10e and a network. Furthermore, the program 10h may be downloaded from the network to the computer 10 through the communication IF device 10e and be stored in the storing device 10d.

The IO device 10f may include one or both of an input device and an output device. Examples of the input device include a keyboard, a mouse, and a touch panel. Examples of the output device include a monitor, a projector, and a printer. The IO device 10f may include, for example, a touch panel that integrates an input device and an output device with each other. The output device may be connected to the graphic processing device 10b.

The reader 10g is an example of a reader that reads information of data and programs recorded on a recording medium 10i. The reader 10g may include a connecting terminal or device to which the recording medium 10i may be connected or inserted. Examples of the reader 10g include an applying adapter conforming to, for example, Universal Serial Bus (USB), a drive apparatus that accesses a recording disk, and a card reader that accesses a flash memory such as an SD card. The program 10h may be stored in the recording medium 10i. The reader 10g may read the program 10h from the recording medium 10i and store the read program 10h into the storing device 10d.

Examples of the recording medium 10i illustratively include a non-transitory computer-readable recording medium such as a magnetic/optical disk, and a flash memory. Examples of the magnetic/optical disk include a flexible disk, a Compact Disc (CD), a Digital Versatile Disc (DVD), a Blu-ray disk, and a Holographic Versatile Disc (HVD). Examples of the flash memory include a semiconductor memory such as a USB memory and an SD card.

The HW configuration of the computer 10 described above is exemplary. Accordingly, the computer 10 may appropriately undergo increase or decrease of HW devices (e.g., addition or deletion of arbitrary blocks), division, integration in an arbitrary combination, or addition or deletion of the bus.

Examples of Functional Configuration:

As illustrated in FIG. 1, the information processing device 11 (sic, correctly, "1") may exemplarily have functions as a 3D structure calculating processor 101 a graph AI calculating processor 102, a graph AI 103, a statistical feature processor 104, a chemical parameter calculating processor 105, a 3D structure feature processor 106, a graph data shaping processor 107, a chemical feature processor 108, an amino-acid sequence calculating processor 109, and a regression model 110. These functions may be implemented by the hardware of a computer 10 (see FIG. 3).

The 3D structure calculating processor 101 analyzes the three-dimensional (3D) structure of the protein of a virus. When an amino-acid sequence of a virus is input, the 3D structure calculating processor 101 analyzes the three-dimensional structure of the amino acid (protein). The 3D structure calculating processor 101 outputs the 3D structure information of the amino acid as a result of the analysis. The 3D structure information of an amino acid may include, for example, a coordinate of each atom.

The function of the 3D structure calculating processor 101 may be realized by using a known structure calculating tool for a protein. For example, AlphaFold2 may be used as a structure calculating tool for a protein, for example.

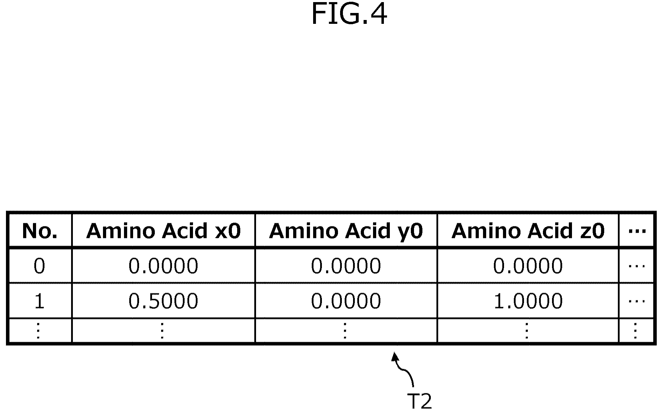

FIG. 4 is a diagram illustrating amino-acid 3D structure information that the 3D structure calculating processor 101 outputs in the information processing device 1 according to the one embodiment.

In FIG. 4, the amino-acid 3D structure information is represented in a data table format. Hereinafter, the amino-acid 3D structure information is sometimes represented by attaching thereto the reference sign T2.

In the amino-acid 3D structure information T2 illustrated in FIG. 4, a coordinate value of each amino acid is associated with the "No." that specifies a virus.

The coordinate value of each amino acid includes x, y, and z coordinate values. In FIG. 4, the coordinate of the amino acid having the amino-acid umber 0 is represented by attaching an amino-acid number 0 to each of the amino acid x, the amino acid y, and the amino acid z, for example.

Also in the amino-acid 3D structure information T2, the order of the amino-acid names may be, for example, from the beginning to the end in the order of the peptide bond.

The amino-acid 3D structure information T2 that the 3D structure calculating processor 101 outputs may be stored in, for example, a predetermined storing region of the memory 10c or the storing device 10d.

The 3D structure feature processor 106 generates a 3D structure feature based on the amino-acid 3D structure information T2 that the 3D structure calculating processor 101 generates. A 3D structure feature represents a feature of a 3D structure and corresponds to a first feature related to the 3D structure of a viral protein.

The 3D structure feature processor 106 may generate a feature of the 3D structure using a known feature converting scheme. For example, the 3D structure feature processor 106 may convert a feature using schemes such as SVR (Support Vector Regression), NN (Neural Network), and PCA (Principal Component Analysis).

The 3D structure feature processor 106 may calculate a 3D structure feature $f_{cube}(t)$ based on the following equation (1).

$$f_{cube}(t) = F_{cube}(a(t), am(t), t) \qquad (1)$$

wherein $f_{cube}(t) = [f_{cube,i}(t)]_{i=1}^N$, the 3D structure feature vector at time t is represented by $f_{cube,i}(t)$ ER ($1 \leq i \leq N$)

The symbol N represents the length of the amino-acid sequence, i.e., the dimension number of the vector. Alternatively, the symbol N represents the dimension number after the sequence is processed into a constant length in a known scheme of generating a fixed vector.

The symbol t represents time. The symbol t is extracted from the amino-acid sequence and antigen cluster name information and may be sampled in a unit of year, for example.

The term a(t) is represented by a $(t)=[a_i(t)]_{i=1}^N$, and is a vector of an amino-acid sequence at time t ($a_i(t) \in \{j \in Z|$ $1 \leq j \leq 20\}$, $1 \leq i \leq N$: a number associated with each of the 20 types of amino acids).

The term am(t) is edge information (e.g., adjacency matrix) of the amino-acid 3D structure at time t. The simplest conceivable $F_{cube}$ is identity mapping (outputting a feature without any modification).

A 3D structure feature calculated by the 3D structure feature processor 106 may be stored in, for example, a predetermined storing region of the memory 10c or the storing device 10d.

The chemical parameter calculating processor 105 generates a chemical parameter for each amino acid included in a virus on the basis of the amino-acid 3D structure information that the 3D structure calculating processor 101 generates. Examples of the chemical parameter may be an electric charge or an exposed surface area. The chemical parameter calculating processor 105 may calculate the exposed surface area or a charge for each amino acid.

The chemical parameter calculating processor 105 may generate a chemical parameter using various known techniques. For example, the chemical parameter calculating processor 105 may calculate a feature of the exposed surface area and the like using a known molecular dynamics simulator.

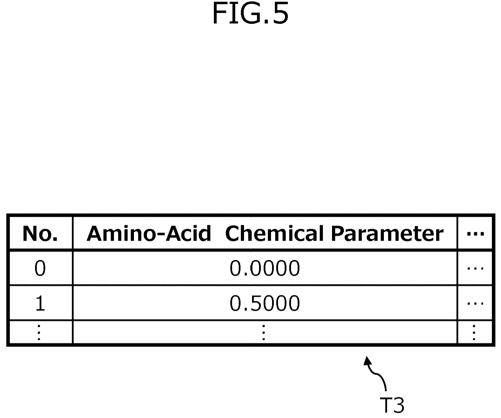

FIG. 5 is a diagram illustrating chemical parameter information generated by the chemical parameter calculating processor 105 in the information processing device 1 according to the one embodiment.

In FIG. 5, this chemical parameter information is represented in a data table format including multiple chemical parameters. Hereinafter, the chemical parameter information is sometimes represented by attaching thereto the reference sign T3.

The chemical parameter information T3 illustrated in FIG. 5 associates values of a chemical parameter of multiple amino acids with a "No." that specifies a virus.

In FIG. 5, for example, the amino-acid chemical parameter 0, which attaches the amino-acid number 0 to the amino-acid chemical parameter, represents the chemical parameter of an amino-acid having the amino-acid number 0.

Also in the chemical parameter information T3, the order of the amino-acid names may be, for example, from the beginning to the end in the order of the peptide bond.

In addition, the chemical parameter calculating processor 105 may generate multiple types of chemical parameter for each amino acid.

The chemical parameter information generated by the chemical parameter calculating processor 105 may be stored in, for example, a predetermined storing region of the memory 10c or the storing device 10d.

The chemical feature processor 108 generates a chemical feature based on the chemical parameter generated by the chemical parameter calculating processor 105. The chemical feature represents a feature of a chemical parameter and corresponds to the second feature related to a property (e.g., exposed surface area) originated from the 3D structure.

The chemical feature processor 108 may generate a feature of the chemical parameter using a known feature conversion scheme. For example, the chemical feature processor 108 may perform feature conversion using the schemes such as SVR, NN, and PCA. Generating a chemical feature based on chemical parameter may be referred to as feature conversion.

The chemical feature processor 108 may calculate chemical feature $f_{chem}(t)$ based the following equation (2).

$$f_{chem}(t) = F_{chem}(a(t), am(t), t) \qquad (2)$$

where, $f_{chem}(t) = [f_{chem,i}(t)]_{i=1}^N$, the chemical feature vector at time t is represented by $f_{chem,i}(t)$ ER ($1 \leq i \leq N$)

The simplest conceivable $F_{chem}$ is identity mapping (outputting a feature without any modification).

The chemical feature calculated by the chemical feature processor 108 may be stored in, for example, a predetermined storing region of the memory 10c or the storing device 10d.

The graph data shaping processor 107 generates graph information based on the amino-acid 3D structure information T2 generated by the 3D structure calculating processor 101 and the chemical parameter information T3 generated by the chemical parameter calculating processor 105. The graph information may be also referred to as graph data.

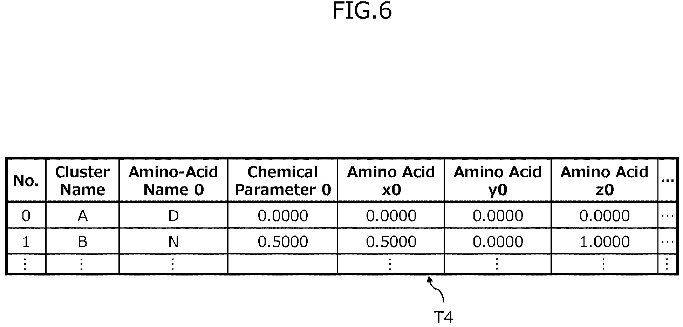

FIG. 6 is a diagram illustrating the graph information in the information processing device 1 according to the one embodiment.

In FIG. 6, the graph information is represented in a data table format. Hereinafter, the graph information is sometimes represented by attaching thereto the reference sign T4.

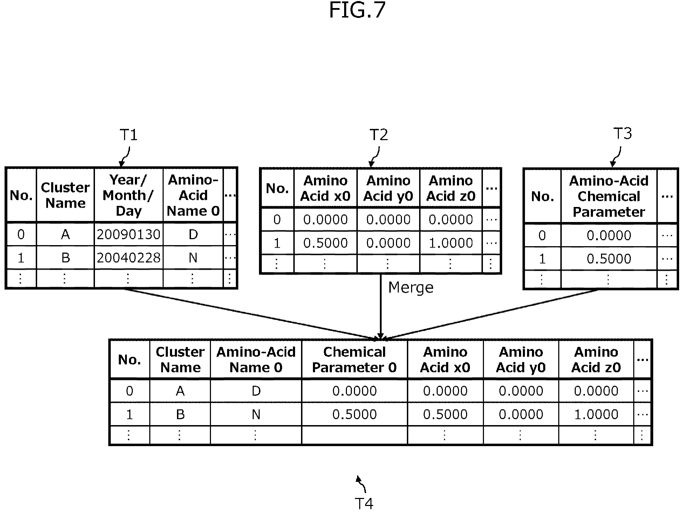

FIG. 7 is a diagram illustrating a process performed by the graph data shaping processor 107 in the information processing device 1 according to the one embodiment.

The graph data shaping processor 107 generates a graph information T4 by merging (combining) the amino-acid sequence and antigen cluster name information T1, the amino-acid 3D structure information T2, and the chemical parameter information T3.

In generating the graph information T4, the graph data shaping processor 107 may merge the amino-acid sequence and antigen cluster name information T1, the amino-acid 3D structure information T2, and the chemical parameter information T3 on the basis of "No.", which specifies a virus.

The graph AI calculating processor 102 creates (shapes), based on the graph information T4 generated by the graph data shaping processor 107, data (graph AI input information T5) to be input into the graph AI 103.

The graph AI calculating processor 102 generates the graph AI input information T5 by converting information about multiple viruses included in the generated graph information T4 into data in the formats that the graph AI 103 can process.

The graph AI calculating processor 102 trains (machine learning) the graph AI 103 using the graph AI input information T5 in the training phase.

Here, the graph AI 103 is a machine learning model that performs graph-based relational learning, and achieves graph classification (class classification).

A graph is configured to include an aggregation of nodes and an aggregation of edges between the above nodes. It can be said that the graph is a mathematical model characterized by the nodes and the edges.

When the graph is applied to a virus, the amino acids correspond to the nodes, and bindings between the amino acids correspond to the edges. Binding between the amino acids may be, for example, the peptide bond, or may alternatively be binding by electrostatic force, or others.

The graph AI 103 performs graph classification based on the information of these graph and these edges. In this classification, the amino-acid 3D structure may be used as an explanatory variable, and the antigen cluster name may be used as a response variable.

The graph classification may carry out the classification on the basis of the parameter of each node serving as a node attribute.

In order to cause the graph AI 103 to perform the graph classification, the edges have to be explicitly provided to the graph AI 103. For this purpose, the graph AI calculating processor 102 assumes that adjacent amino acids have an edge on the basis of the amino-acid sequence. Furthermore, amino acids within a certain distance under the influence of, for example, electrostatic force are assumed to have an edge.

The function of the graph AI 103 can be achieved by using a known scheme. For example, the function of the graph AI 103 may be achieved by Deep Tensor (registered trademark).

On the basis of the graph information T4, the graph AI calculating processor 102 generate the graph AI input information T5 by arranging, for each edge in an amino-acid sequence forming a virus, the attributes of two amino acids that the edge binds to each other in a unit of binding. Hereinafter, the two amino acids that an edge binds to each other may be referred to as an amino-acid pair. The amino acid at the beginning of the edge of an amino-acid pair may be referred to as a starting node, and the amino acid at the end of the edge may be referred to as an end node.

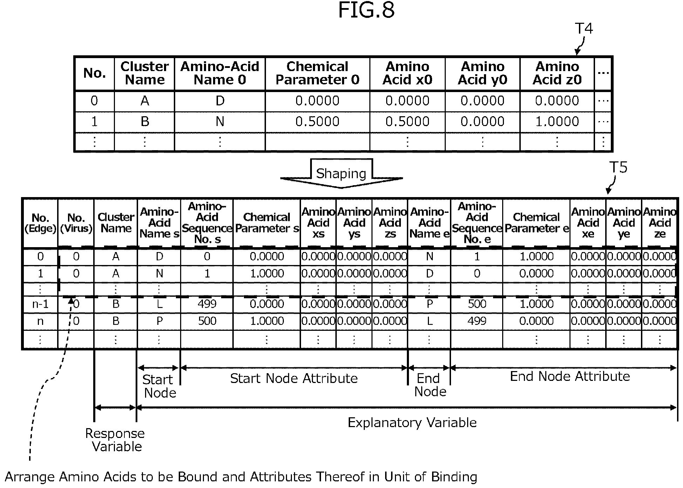

FIG. 8 is a diagram illustrating the graph AI input information T5 in the information processing device 1 according to the one embodiment.

FIG. 8 illustrates the graph information T4 illustrated in FIG. 6 and the graph AI input information T5 that the graph AI calculating processor 102 generates on the basis of the graph information T4.

The graph AI input information T5 illustrated in FIG. 8 associates information of the amino-acid pair that an edge binds to each other with the "No.", which that specifies the edge.

The information of an amino-acid pair includes "No." that specifies virus, a cluster name, and amino-acid name, an amino-acid sequence number, a chemical parameter, and coordinate values (x, y, z) of at the starting node and the end node. In the example illustrated in FIG. 8, the symbol "s" is attached to the end of each piece of information of the starting node, and the symbol "e" is attached to the end of each piece of information of the end node.

Accordingly, for example, the amino-acid name s represents the starting node and the amino-acid name e represents the end node. In addition, the amino-acid sequence number s, the chemical parameter s, the amino acid xs, the amino-acid name ys, and the amino acid zs represent attribute information (starting node attribute) of the starting node. Similarly, the amino-acid sequence number e, the chemical parameter e, the amino acid xe, the amino-acid name ye and the amino acid ze represent attribute information (end node attribute) of the end node.

In the training phase, the graph AI calculating processor 102 trains the graph AI 103, using the graph AI input information T5 as training data.

In the graph AI input information T5 illustrated in FIG. 8, the cluster name is used as a response variable in the training phase of the graph AI 103. The amino-acid name s, the amino-acid name e, the starting node attributes, and the end node attributes are used as explanatory variables in the training phase of the graph AI 103.

The graph AI 103 may be a Deep Neural Network (DNN) that includes multiple hidden layers between an input layer and an output layer.

For example, a NN executes a process (forward propagation process) in the forward direction in which process the information obtained by the calculations is sequentially transmitted from the input side to the output side by inputting input data into an input layer and sequentially executing predetermined calculations in the hidden layers composed of a convolutional layer, a pooling layer, or the like. After executing the processing in the forward direction, the NN executes a process (backward propagation process) in the backward direction in which process a parameter to be used in the process in the forward direction is determined in order to reduce the value of an error function obtained from output data (result of the graph classification) output from the output layer and the correct answer data (cluster name). Then, an updating process that updates a variable such as a weight is executed based on the result of the backward propagation process. For example, a gradient descent method may be used as an algorithm to determine the updating width of a weight to be used in the calculation of the backward propagation process.

Furthermore, in the predicting phase, the graph AI calculating processor 102 causes the graph AI 103 to execute the graph classification using the graph AI input information T5 and thereby predict (infer) the cluster name.

The amino-acid name s, the amino-acid name e, starting node attributes and the end node attributes included in the graph AI input information T5 illustrated in FIG. 8 are input into the graph AI 103.

In the predicting phase, the cluster name is not included in the amino-acid sequence and antigen cluster name information T1 described above. Accordingly, the cluster name is not included in the graph AI input information T5 input into the graph AI 103 in the predicting phase.

The graph AI calculating processor 102 inputs a feature (3D structure feature) related to the 3D structure of a protein of a virus and a feature (chemical feature) related to a property originated from the 3D structure into the graph AI 103 and causes the graph AI 103 to predict an amino acid.

In addition, in the training phase and the predicting phase, the graph AI calculating processor 102 inputs the graph AI input information T5 into the graph AI 103 and causes the graph AI 103 to carry out graph classification (class classification) and then calculate statistical information.

The statistical information may be, for example, a contribution (contribution score, node contribution) for obtaining a result of the prediction when the graph AI 103 performs the graph classification. The statistical information may be referred to as a statistic. The graph AI calculating processor 102 obtains a statistic for each amino acid contained in the virus.

This means that, for the prediction, the graph AI calculating processor 102 obtains a feature (statistical feature) based on a contribution to prediction of the respective amino acids included in the protein.

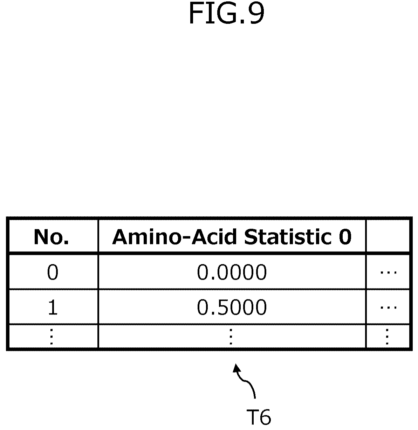

FIG. 9 is a diagram illustrating the statistical information in the information processing device 1 according to the one embodiment.

In FIG. 9, multiple pieces of the statistical information are represented in a data table format. Hereinafter, the statistical information is sometimes represented by attaching thereto the reference sign T6.

The statistical information T6 illustrated in FIG. 8 associates values of the statistical information of multiple amino acids with the "No." that specifies a virus.

In FIG. 9, for example, the amino-acid statistical information 0, which attaches the amino-acid number 0 to the amino-acid statistical information, represents the amino-acid statistical information of an amino-acid having the amino-acid number 0.

Also in the statistical information T6, the order of the amino-acid names may be, for example, from the beginning to the end in the order of the peptide bond.

The statistical information that the graph AI calculating processor 102 generates may be stored in, for example, a predetermined storing region of the memory 10c or the storing device 10d.

In the graph AI (graph AI 103), the contribution is obtained for each 3D structure and for each amino acid. For the above, the graph AI calculating processor 102 may obtain a sample mean of contribution in predetermined units of, for example, a cluster, a year, and an amino acid, and may use the obtained sample mean as the statistical information.

The result of the prediction that the graph AI calculating processor 102 causes the graph AI 103 to carry out and the values of the statistical information that the graph AI calculating processor 102 causes the graph AI 103 to calculate may be stored in, for example, a predetermined storing region of the memory 10c or the storing device 10d.

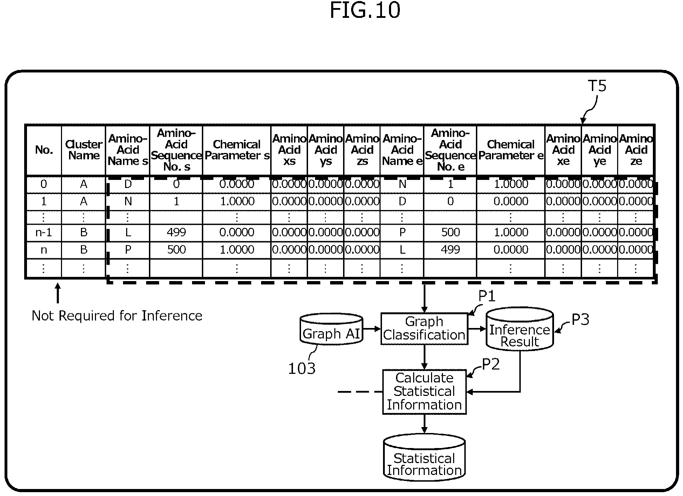

FIG. 10 is a diagram illustrating a process in the predicting phase of the graph AI calculating processor 102 of the information processing device 1 according to the one embodiment.

In predicting phase, the graph AI calculating processor 102 inputs the graph AI input information T5 into the graph AI 103 and causes the graph AI 103 to perform the graph classification (see the reference sign P1). In addition, the graph AI calculating processor 102 obtains the statistical information (contribution) that the graph AI 103 calculates (see the reference sign P2).

In the predicting phase, the graph AI calculating processor 102 shifts the values included in the graph AI input information T5 and confirms how the result of the inference changes (see the reference sign P3). If the result of the inference improves, the graph AI calculating processor 102 may process the graph AI input information T5 to reflect the change.

The statistical feature processor 104 generates the statistical feature based on the statistical information T6 that the graph AI calculating processor 102 causes the graph AI 103 to calculate. The statistical feature represents a feature of the statistical information (contribution).

The statistical feature corresponds to a third feature obtained on the basis of the statistical information (contribution) being obtained by prediction of each of amino acids included in a protein corresponding to input data, which includes the three-dimensional feature and the chemical feature structure, which prediction is performed by inputting the input data into the graph AI 103 (machine learning model).

The statistical feature processor 104 may generate the statistical feature using a known feature conversion scheme. For example, the statistical feature processor 104 may perform feature conversion using the schemes such as SVR, NN, and PCA.

The statistical feature processor 104 may calculate the statistical feature $f_{stat}(t)$ on the basis of the following equation (3).

$$f_{stat}(t) = F_{stat}\ (a(t),\ am(t),\ t)\ (3)$$

wherein, $f_{stat}(t) = [f_{stat,\ i}(t)]_{i=1}^N$, the statistical feature vector at time t is represented by $f_{stat,\ i}(t)$ ER $(1 \leq i \leq N)$

The simplest conceivable $F_{stat}$ is identity mapping (outputting a feature without any modification).

The statistical feature that the statistical feature processor 104 calculates may be stored in, for example, a predetermined storing region of the memory 10c or the storing device 10d.

The amino-acid sequence calculating processor 109 predicts an amino-acid sequence of the virus after the mutation, using a regression model 110.

The amino-acid sequence calculating processor 109 trains the regression model 110 in the training phase, and causes the regression model 110 to predict the amino-acid sequence after the mutation in predicting phase.

The amino-acid sequence calculating processor 109 predicts the amino-acid sequence after the mutation on the basis of the 3D structure feature $f_{cube}(t)$ calculated by the 3D structure feature processor 106, the chemical feature $f_{chem}(t)$ calculated by the chemical feature processor 108, and the statistical feature $f_{stat}(t)$ calculated by the statistical feature processor 104. At this time, the amino-acid sequence calculating processor 109 also uses edge information am(t) of the amino-acid sequence.

The regression model 110 may achieve regression by using a scheme such SVR, NN, GA (Genetic Algorithms), a time series analysis.

Alternatively, the regression model 110 may be a deep neural network (DNN) that includes multiple hidden layers between the input-layer and the output-layer.

The amino-acid sequence calculating processor 109 trains the regression model 110, which predicts an amino-acid sequence of the virus after mutation using the 3D structure feature, the chemical feature, and the statistical feature as the input features (explanatory variables), and predicts mutation using the regression model 110 after being subjected to the training.

The regression model 110 obtains the amino-acid sequence after the mutation using the following equation (4).

$$a\ (t+n\Delta t) = F_a\{f_{cube}(t),\ f_{chem}(t),\ f_{stat}(t),\ am\ (t),\ ...,\ f_{cube}(t-n\Delta t),\ f_{chem}(t-n\Delta t),\ f_{stat}\ (t-n\Delta t),\ am\ (t-n\Delta t),\ t\}\ (4) \qquad (4)$$

$F_a$ may be an SVR, an LSTM, GA, or a time series analysis. The symbol t represents the present time. The term $t-\Delta t$ represents the previous time before by $\Delta t$ from the present time. The term $t+\Delta t$ represents the future time after by $\Delta t$ from the present time. Thus, $a(t+n\Delta t)$ represents the future amino-acid sequence, i.e., amino-acid sequence after mutation.

The amino-acid sequence calculating processor 109 trains the regression model 110 using at least the chemical feature (second feature) and the statistical feature (third feature) as the explanatory variables (input features) in the training phase.

In addition, in the training phase, the amino-acid sequence calculating processor 109 may also use the 3D structure feature (first feature) in addition to the chemical feature and the statistical feature as an explanatory variable (input feature).

Here, the regression calculation assumes that the data lengths (dimension when vectorized) of the input and output are fixed. However, the length of an amino-acid sequence varies with virus. For this reason, it is necessary to convert different dimensions into a fixed dimension.

For this purpose, in inputting the 3D structure feature, the chemical feature, and the statistical feature into the regression model 110, the amino-acid sequence calculating processor 109 performs a process of converting the dimensions of these features into the fixed dimension that the regression model 110 can handle.

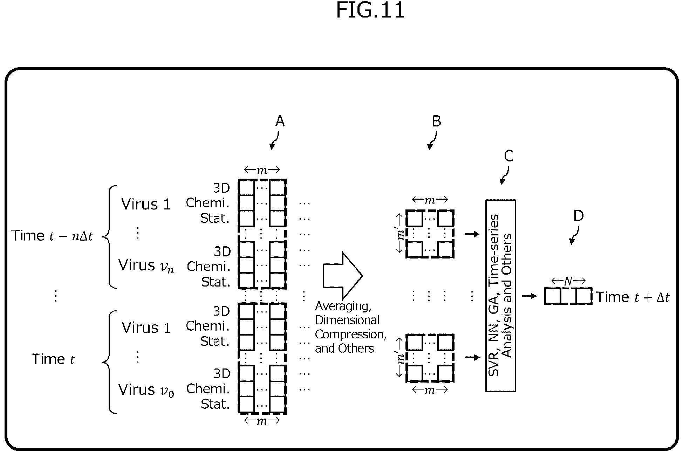

FIG. 11 is a diagram illustrating a process performed by the amino-acid sequence calculating processor 109 of the information processing device 1 according to the one embodiment.

In FIG. 11, the reference sign A represents a matrix (feature matrix) of features extracted from the graph AI input information T5.

The amino-acid sequence calculating processor 109 generates a feature matrix by extracting features of multiple viruses at respective times.

For example, the amino-acid sequence calculating processor 109 generates multiple $m \times (3 \times v_0)$ feature matrices by sequentially extracting units of m features from the head of amino-acid sequence included in each of the viruses 1 to $v_0$ at time t.

The rectangles expressed in dashed lines include the 3D structure feature, the chemical feature, and the statistical feature of the respective viruses.

For example, the amino-acid sequence calculating processor 109 may generate the feature matrices by conversion to a fixed dimension using, for example, a projection method such as m-gram, EG-PSSM, GDPC-PSMM, and ER-PSSM. For example, m-gram may use units of m features sequentially from the head of an amino-acid sequence as vectors (imitating natural-language processes).

Further, the amino-acid sequence calculating processor 109 performs compression (dimensional compression) on the generated feature matrices, and thereby generates matrices (low-dimensional matrices) each having a lower dimension than the feature matrices. Accordingly, the amino-acid sequence calculating processor 109 shapes the respective feature matrices into data that can be input into the regression.

The amino-acid sequence calculating processor 109 may compress the feature matrices by applying, for example, averaging or dimensional compression.

FIG. 11 illustrates an example in which the amino-acid sequence calculating processor 109 compresses the $m \times (3 \times v_0)$ feature matrices and generates $m \times m'$ feature matrices (see the reference sign B).

The amino-acid sequence calculating processor 109 vectorizes the generated $m \times m'$ feature matrices, inputs the generated vectors into the regression model 110 (see the reference sign C), and outputs the amino-acid sequence after the mutation. In the example illustrated in FIG. 11, an amino-acid sequence of N rows at $t + \Delta t$ is output (see the reference numeral D).

The circumstance where a single amino-acid sequence is not determined because multiple outputs are obtained from the regression model 110 in the training phase means that various viruses exist in the time interval ($\Delta t$) in the phylogenetic tree. In such a cases, referring to the phylogenetic tree, the time interval ($\Delta t$) of the hierarchical level may be adjusted such that each interval includes a single generation (mutation). The above may be achieved by decreasing each interval ($\Delta t$) or adjusting the number of steps (n). This allows the regression model 110 to predict one amino-acid sequence.

Operation:

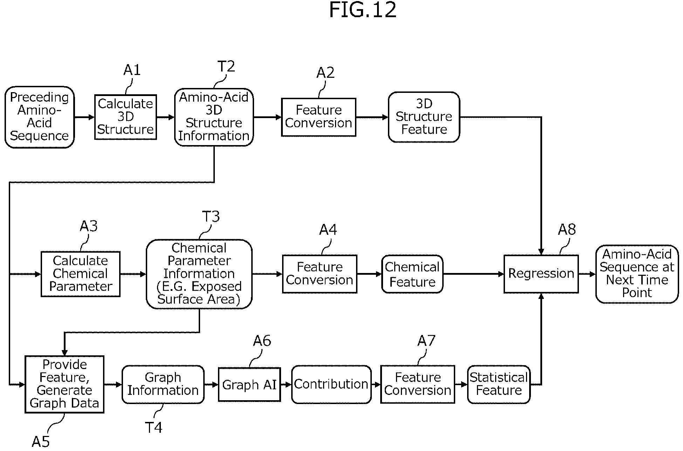

Description will now be made in relation to a process in the training phase in the information processing device 1 according to the one embodiment having the above configuration with reference to a flow chart (Steps A1 to A8) illustrated in FIG. 12.

When preceding amino-acid sequence of the virus is input into the 3D structure calculating processor 101, the 3D structure calculating processor 101 performs the three-dimensional structure analysis of the amino acid in Step A1. The 3D structure calculating processor 101 generates amino-acid 3D structure information T2.

The amino-acid 3D structure information T2 is input into the 3D structure feature processor 106. In Step A2, the 3D structure feature processor 106 generates a 3D structure feature based on the amino-acid 3D structure information T2.

The amino-acid 3D structure information T2 is also input into the chemical parameter calculating processor 105. In Step A3, the chemical parameter calculating processor 105 generates a chemical parameter for each amino acid contained in the virus on the basis of the amino-acid 3D structure information T2, and generates chemical parameter information T3.

The chemical parameter information T3 generated by the chemical parameter calculating processor 105 is input to the chemical feature processor 108. In Step A4, the chemical feature processor 108 generates a chemical feature based on the chemical parameter information T3.

The amino-acid 3D structure information T2 generated by the 3D structure calculating processor 101 and the chemical parameter information T3 generated by the chemical parameter calculating processor 105 are also input into the graph data shaping processor 107. In Step A5, the graph data shaping processor 107 generates graph information T4 based on the amino-acid 3D structure information T2 and the chemical parameter information T3.

The graph information T4 generated by the graph data shaping processor 107 is input into the graph AI 103. On the basis of the graph information T4, the graph AI calculating processor 102 generates graph AI input information T5 by arranging, for each edge in an amino-acid sequence forming a virus, the attributes of two amino acids that the edge binds to each other in a unit of binding.

The graph AI calculating processor 102 trains the graph AI 103, using the graph AI input information T5 as training data. The graph AI calculating processor 102 causes the graph AI 103 to calculate statistical information (contribution) and generates statistical information T6.

The statistical information T6 generated by the graph AI calculating processor 102 is input into the statistical feature processor 104. In Step A7, the statistical feature processor 104 generates a statistical feature based on the statistical information T6.

The 3D structure feature generated by the 3D structure feature processor 106, the chemical feature generated by the chemical feature processor 108, and the statistical feature generated by the statistical feature processor 104 are input into the amino-acid sequence calculating processor 109.

In Step A8, the amino-acid sequence calculating processor 109 converts the 3D structure feature, the chemical feature, and the statistical feature into the fixed dimension and then inputs the converted features to the regression model 110 to predict the amino-acid sequence.

The amino-acid sequence calculating processor 109 compares the predicted amino-acid sequence with the correct answer data (amino-acid sequence after the mutation). With reference to the result of this comparison, the amino-acid sequence calculating processor 109 performs a process (backward propagation process) in the backward direction for determining a parameter to be used in a process in the forward direction in order to reduce the value of an error function to be obtained. The amino-acid sequence calculating processor 109 then performs the updating process that updates a variable such as a weight on the basis of the result of the backward propagation process.

The information processing device 1 of the present embodiment configured as the above carries out the same process (Step A1 to A8) also in the predicting phase.

However, the present amino-acid sequence of the virus is input into the 3D structure calculating processor 101. In Step A1, the 3D structure calculating processor 101 performs the three-dimensional structure analysis on the amino acid in response. The 3D structure calculating processor 101 generates amino-acid 3D structure information T2.

The amino-acid 3D structure information T2 is input into the 3D structure feature processor 106. In Step A2, the 3D structure feature processor 106 generates a 3D structure feature (feature conversion) based on the amino-acid 3D structure information T2.

The amino-acid 3D structure information T2 is also input into the chemical parameter calculating processor 105. In Step A3, the chemical parameter calculating processor 105 generates a chemical parameter for each amino acid contained in the virus on the basis of the amino-acid 3D structure information T2, and generates chemical parameter information T3.

The chemical parameter information T3 generated by the chemical parameter calculating processor 105 is input into the chemical feature processor 108. In Step A4, the chemical feature processor 108 generates (feature conversion)a chemical feature based on the chemical parameter information T3.

The amino-acid 3D structure information T2 generated by the 3D structure calculating processor 101 and the chemical parameter information T3 generated by the chemical parameter calculating processor 105 are also input into the graph data shaping processor 107. In Step A5, the graph data shaping processor 107 generates graph information T4 based on the amino-acid 3D structure information T2 and the chemical parameter information T3.

The graph information T4 generated by the graph data shaping processor 107 is input into the graph AI 103. On the basis of the graph information T4, the graph AI calculating processor 102 generates graph AI input information T5 by arranging, for each edge in an amino-acid sequence forming a virus, the attributes of two amino acids that the edge binds to each other in a unit of binding.

The graph AI calculating processor 102 inputs the graph AI input information T5 into the graph AI 103 and causes the graph AI 103 to calculate the statistical information (contribution) and generate the statistical information T6.

The statistical information T6 generated by the graph AI calculating processor 102 is input into the statistical feature processor 104. In Step A7, the statistical feature processor 104 generates a statistical feature (feature conversion) based on the statistical information T6.

The 3D structure feature generated by the 3D structure feature processor 106, the chemical feature generated by the chemical feature processor 108, and the statistical feature generated by the statistical feature processor 104 are input into the amino-acid sequence calculating processor 109.

In Step A8, the amino-acid sequence calculating processor 109 converts the 3D structure feature, the chemical feature, and the statistical feature into the fixed dimension and then inputs the converted features to the regression model 110 to predict the amino-acid sequence after the mutation.

The amino-acid sequence that the regression model 110 outputs in the predicting phase may be used as training data in the subsequent training phase.

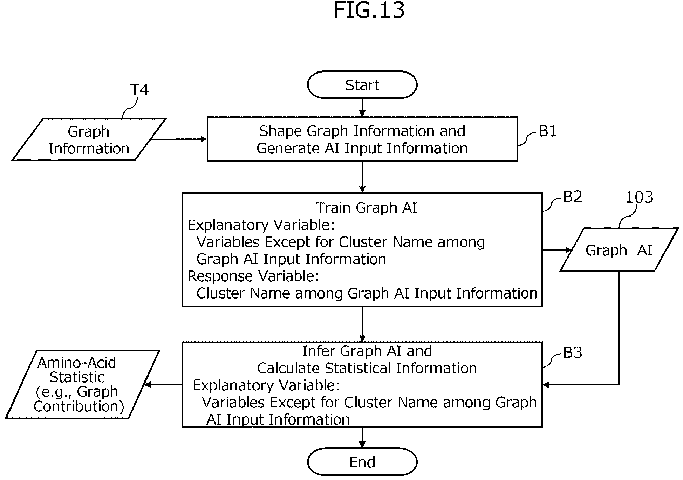

Next, description will now be made in relation to a process of the graph AI calculating processor 102 of the information processing device 1 according to the one embodiment having the above configuration with reference to a flow chart (Steps B1 to B3) illustrated in FIG. 13.

In Step B1, the graph AI calculating processor 102 shapes the graph information T4 generated by the graph data shaping processor 107 to generate graph AI input information T5.

In the training phase, the graph AI calculating processor 102 trains the graph AI 103, using the generated graph AI input information T5 (step B2).

At this time, the graph AI calculating processor 102 uses the graph AI input information T5 except for the cluster name as explanatory variables, and uses the cluster name as the response variable.

In the predicting phase, the graph AI calculating processor 102 inputs the graph AI input information T5 into the graph AI 103 and causes the graph AI 103 to predict (infer) the cluster name (Step B3). At this time, the graph AI calculating processor 102 uses graph AI input information T5 except for the cluster name as the explanatory variables.

In addition, the graph AI calculating processor 102 causes the graph AI 103 to calculate the statistical information. Then, the process ends.

Effect:

As the above, in the information processing device 1 according to the one embodiment, in the training phase that trains the regression model 110, which predicts the amino-acid sequence of a virus after mutation, the graph AI calculating processor 102 causes the graph AI 103 to carry out graph classification (prediction), using the 3D structure feature (first feature) related to the 3D structure of the protein of the virus and the chemical feature (second feature) related to a property originated from the 3D structure as inputs.

The statistical feature processor 104 calculates the statistical feature (third feature) based on statistical information (contribution) calculated along with the graph classification.

Then, the amino-acid sequence calculating processor 109 trains the regression model 110 using at least the chemical feature and the statistical feature as the input features.

This reflects the 3D structure of the protein of the virus in the regression model 110. Accordingly, since the regression model 110 can perform prediction of the virus mutation considering the property unique to the 3D structure of the protein of the virus in the predicting phase, the accuracy in the prediction can be enhanced.

Since the amino-acid sequence calculating processor 109 trains the regression model 110 using at least the 3D structure feature in addition to the chemical feature and the statistical feature as the input features, the 3D structure of the protein of the virus can be further reflected in the regression model 110. This enhances the accuracy in the prediction of mutation of a virus, considering the property unique to the 3D structure of the protein of the virus.

A protein is composed of multiple amino acids bound via peptide bond, and an amino-acid sequence is a sequence of the amino acids in this order of the binding. However, amino acids distant from each other in an amino-acid sequence may be bound to each other via, for example, electrostatic force, which provides a unique shape and a unique property. This means that the same amino-acid sequence may have different features due to such a unique shape and a unique property.

In the present information processing device 1, the accuracy in the prediction can be enhanced by prediction the mutation of the virus from the feature based the 3D structure of the protein.

In inputting the 3D structure feature, the chemical feature, and the statistical feature into the regression model 110, the amino-acid sequence calculating processor 109 performs a process of converting the dimensions of these features into the fixed dimension that the regression model 110 can handle. This enables the regression model 110 to perform prediction using the 3D structure feature, the chemical feature and the statistical feature as the input.

Furthermore, at this time, the Fa can be easily calculated and the accuracy in the precision can be enhanced by regression such as SVR, NN, and PCA, dimension compression, conversion to a fixed-dimensional vector such as m-gram, and generation of a fixed-dimensional vector.

Miscellaneous:

The disclosed techniques are not limited to the embodiment described above, and may be variously modified without departing from the scope of the present embodiment. The respective configurations and processes of the present embodiment can be selected, omitted, and combined according to the requirement.

For example, the above-described embodiment uses a contribution as the statistical information. The statistical information is not limited to this, and may alternatively be information except for the contribution.

The present embodiment can be executed or produced by those ordinary skilled in the art referring to the above disclosure.

1       information processing device
10      computer
10a     processor

10b    graphic processing device
10c    memory
10d    storing device
10e    IF device
10f    IO device
10g    reader
10h    program
10i    recording medium
10j    bus
101    3D structure calculating processor
102    graph AI calculating processor
103    graph AI
104    statistical feature processor
105    chemical parameter calculating processor
106    3D structure feature processor
107    graph data shaping processor
108    chemical feature processor
109    amino-acid sequence calculating processor
110    regression model
T1    amino-acid sequence and antigen cluster name information
T2    amino-acid 3D structure information
T3    chemical parameter information
T4    graph information
T5    graph AI input information
T6    statistical information

**Claims**

1.  An information processing program for causing a computer to execute a process comprising:

    obtaining a third feature based on statistical information, the statistical information being obtained by prediction of each of amino acids included in a protein corresponding to input data including a first feature related to a three-dimensional structure of a protein of a virus and a second feature related to a property originated from the three-dimensional structure, the prediction being performed by inputting the input data into a machine-learning model, and

    training a regression model that predicts an amino-acid sequence of the virus after mutation using the second feature and the third feature as an input feature.

2.  The information processing program according to claim 1, wherein
    the training the regression model uses the first feature as the input feature in addition to the second feature and the third feature.

3.  The information processing program according to claim 1 or 2, wherein the process further comprises:

    generating amino-acid three-dimensional structure information by three-dimensional analysis of amino acids of the protein; and
    calculating a feature of the three-dimensional structure by feature conversion on the amino-acid three-dimensional structure information.

4.  The information processing program according to claim 3, wherein the process further comprises:

    calculating, based on the amino-acid three-dimensional structure information, chemical parameter information of each of amino acids included in the protein; and
    generating a chemical feature by feature conversion on the chemical parameter information.

5.  The information processing program according to claim 1, wherein the process further comprises:
    converting dimensions of the second feature and the feature to a fixed dimension suitable for the regression model at least before the second feature and the third feature are input into the regression model.

6. A computer-implemented method for processing information, the method comprising:

    obtaining a third feature based on statistical information, the statistical information being obtained by prediction of each of amino acids included in a protein corresponding to input data including a first feature related to a three-dimensional structure of a protein of a virus and a second feature related to a property originated from the three-dimensional structure, the prediction being performed by inputting the input data into a machine-learning model, and

    training a regression model that predicts an amino-acid sequence of the virus after mutation using the second feature and the third feature as an input feature.

7. The computer-implemented method according to claim 6, wherein
    the training the regression model uses the first feature as the input feature in addition to the second feature and the third feature.

8. The computer-implemented method according to claim 6 or 7, further comprising:

    generating amino-acid three-dimensional structure information by three-dimensional analysis of amino acids of the protein; and

    calculating a feature of the three-dimensional structure by feature conversion on the amino-acid three-dimensional structure information.

9. The computer-implemented method according to claim 8, further comprising:

    calculating, based on the amino-acid three-dimensional structure information, chemical parameter information of each of amino acids included in the protein; and

    generating a chemical feature by feature conversion on the chemical parameter information.

10. The computer-implemented method according to claim 6, further comprising:
    converting dimensions of the second feature and the feature to a fixed dimension suitable for the regression model at least before the second feature and the third feature are input into the regression model.

11. An information processing device comprising

    a controller configured to execute a process comprising

    obtaining a third feature based on statistical information, the statistical information being obtained by prediction of each of amino acids included in a protein corresponding to input data including a first feature related to a three-dimensional structure of a protein of a virus and a second feature related to a property originated from the three-dimensional structure, the prediction being performed by inputting the input data into a machine-learning model, and

    training a regression model that predicts an amino-acid sequence of the virus after mutation using the second feature and the third feature as an input feature.

12. The information processing device according to claim 11, wherein the controller uses the first feature as the input feature in addition to the second feature and the third feature in the training of the regression model.

13. The information processing device according to claim 11 or 12, wherein the controller further

    generates amino-acid three-dimensional structure information by three-dimensional analysis of amino acids of the protein; and

    calculates a feature of the three-dimensional structure by feature conversion on the amino-acid three-dimensional structure information.

14. The information processing device according to claim 13, wherein the controller further

    calculates, based on the amino-acid three-dimensional structure information, chemical parameter information of each of amino acids included in the protein; and

    generates a chemical feature by feature conversion on the chemical parameter information.

15. The information processing device according to claim 11, wherein the controller further converts dimensions of the second feature and the feature to a fixed dimension suitable for the regression model at least before the second feature and the third feature are input into the regression model.

## FIG.1

# FIG.2

| No. | Cluster Name | Year/Month/Day | Amino-Acid Name 0 | ⋯ |
|-----|--------------|----------------|-------------------|-----|
| 0 | Cluster Name | 20090130 | D | ⋯ |
| 1 | Cluster Name | 20040228 | N | ⋯ |
| ⋮ | ⋮ | ⋮ | ⋮ | ⋮ |

T 1

FIG.3

# FIG.4

| No. | Amino Acid x0 | Amino Acid y0 | Amino Acid z0 | ... |
|-----|---------------|---------------|---------------|-----|
| 0 | 0.0000 | 0.0000 | 0.0000 | ... |
| 1 | 0.5000 | 0.0000 | 1.0000 | ... |
| ⋮ | ⋮ | ⋮ | ⋮ | ⋮ |

T2

# FIG.5

| No. | Amino-Acid  Chemical Parameter | ··· |
|-----|-------------------------------|-----|
| 0 | 0.0000 | ··· |
| 1 | 0.5000 | ··· |
| ⋮ | ⋮ | ⋮ |

T3

FIG.6

| No. | Cluster Name | Amino-Acid Name 0 | Chemical Parameter 0 | Amino Acid x0 | Amino Acid y0 | Amino Acid z0 | ... |
|---|---|---|---|---|---|---|---|
| 0 | A | D | 0.0000 | 0.0000 | 0.0000 | 0.0000 | ... |
| 1 | B | N | 0.5000 | 0.5000 | 0.0000 | 1.0000 | ... |
| ⋮ | ⋮ | ⋮ | | ⋮ | ⋮ | ⋮ | ⋮ |

T4

# FIG.7

T1

| No. | Cluster Name | Year/ Month/ Day | Amino- Acid Name 0 | ··· |
|-----|--------------|-------------------|---------------------|-----|
| 0 | A | 20090130 | D | ··· |
| 1 | B | 20040228 | N | ··· |
| ⋮ | ⋮ | ⋮ | ⋮ | ⋮ |

T2

| No. | Amino Acid x0 | Amino Acid y0 | Amino Acid z0 | ··· |
|-----|---------------|---------------|---------------|-----|
| 0 | 0.0000 | 0.0000 | 0.0000 | ··· |
| 1 | 0.5000 | 0.0000 | 1.0000 | ··· |
| ⋮ | ⋮ | ⋮ | ⋮ | ⋮ |

T3

| No. | Amino-Acid Chemical Parameter | ··· |
|-----|-------------------------------|-----|
| 0 | 0.0000 | ··· |
| 1 | 0.5000 | ··· |
| ⋮ | ⋮ | ⋮ |

Merge

| No. | Cluster Name | Amino-Acid Name 0 | Chemical Parameter 0 | Amino Acid x0 | Amino Acid y0 | Amino Acid z0 | ··· |
|-----|--------------|--------------------|----------------------|---------------|---------------|---------------|-----|
| 0 | A | D | 0.0000 | 0.0000 | 0.0000 | 0.0000 | ··· |
| 1 | B | N | 0.5000 | 0.5000 | 0.0000 | 1.0000 | ··· |
| ⋮ | ⋮ | ⋮ | | ⋮ | ⋮ | ⋮ | ⋮ |

T4

## FIG.8

T4

| No. | Cluster Name | Amino-Acid Name 0 | Chemical Parameter 0 | Amino Acid x0 | Amino Acid y0 | Amino Acid z0 | ... |
|---|---|---|---|---|---|---|---|
| 0 | A | D | 0.0000 | 0.0000 | 0.0000 | 0.0000 | ... |
| 1 | B | N | 0.5000 | 0.5000 | 0.0000 | 1.0000 | ... |
| ⋮ | ⋮ | ⋮ | | ⋮ | ⋮ | ⋮ | ⋮ |

Shaping

T5

| No. (Edge) | No. (Virus) | Cluster Name | Amino-Acid Name s | Amino-Acid Sequence No. s | Chemical Parameter s | Amino Acid xs | Amino Acid ys | Amino Acid zs | Amino-Acid Name e | Amino-Acid Sequence No. e | Chemical Parameter e | Amino Acid xe | Amino Acid ye | Amino Acid ze |
|---|---|---|---|---|---|---|---|---|---|---|---|---|---|---|
| 0 | 0 | A | D | 0 | 0.0000 | 0.0000 | 0.0000 | 0.0000 | N | 1 | 1.0000 | 0.0000 | 0.0000 | 0.0000 |
| 1 | 0 | A | N | 1 | 1.0000 | 0.0000 | 0.0000 | 0.0000 | D | 0 | 0.0000 | 0.0000 | 0.0000 | 0.0000 |
| ⋮ | | ⋮ | ⋮ | | ⋮ | ⋮ | ⋮ | ⋮ | ⋮ | | ⋮ | ⋮ | ⋮ | ⋮ |
| n-1 | 0 | B | L | 499 | 0.0000 | 0.0000 | 0.0000 | 0.0000 | P | 500 | 1.0000 | 0.0000 | 0.0000 | 0.0000 |
| n | 0 | B | P | 500 | 1.0000 | 0.0000 | 0.0000 | 0.0000 | L | 499 | 0.0000 | 0.0000 | 0.0000 | 0.0000 |
| ⋮ | | ⋮ | ⋮ | | ⋮ | ⋮ | ⋮ | ⋮ | ⋮ | | ⋮ | ⋮ | ⋮ | ⋮ |

Start Node — Start Node Attribute — End Node — End Node Attribute

Explanatory Variable

Response Variable

Arrange Amino Acids to be Bound and Attributes Thereof in Unit of Binding

# FIG.9

| No. | Amino-Acid Statistic 0 | |
|:---:|:---:|:---:|
| 0 | 0.0000 | ⋯ |
| 1 | 0.5000 | ⋯ |
| ⋮ | ⋮ | ⋮ |

T6

FIG.10

| No. | Cluster Name | Amino-Acid Name s | Amino-Acid Sequence No. s | Chemical Parameter s | Amino Acid xs | Amino Acid ys | Amino Acid zs | Amino-Acid Name e | Amino-Acid Sequence No. e | Chemical Parameter e | Amino Acid xe | Amino Acid ye | Amino Acid ze |
|---|---|---|---|---|---|---|---|---|---|---|---|---|---|
| 0 | A | D | 0 | 0.0000 | 0.0000 | 0.0000 | 0.0000 | N | 1 | 1.0000 | 0.0000 | 0.0000 | 0.0000 |
| 1 | A | N | 1 | 1.0000 | 0.0000 | 0.0000 | 0.0000 | D | 0 | 0.0000 | 0.0000 | 0.0000 | 0.0000 |
| ⋮ | ⋮ | ⋮ | | ⋮ | ⋮ | ⋮ | ⋮ | ⋮ | | ⋮ | ⋮ | ⋮ | ⋮ |
| n-1 | B | L | 499 | 0.0000 | 0.0000 | 0.0000 | 0.0000 | P | 500 | 1.0000 | 0.0000 | 0.0000 | 0.0000 |
| n | B | P | 500 | 1.0000 | 0.0000 | 0.0000 | 0.0000 | L | 499 | 0.0000 | 0.0000 | 0.0000 | 0.0000 |
| ⋮ | ⋮ | ⋮ | | ⋮ | ⋮ | ⋮ | ⋮ | ⋮ | | ⋮ | ⋮ | ⋮ | ⋮ |

T5

Not Required for Inference

Graph AI — 103

Graph Classification — P1

Inference Result — P3

Calculate Statistical Information — P2

Statistical Information

FIG.11

FIG.12

# FIG.13

T4

```
┌──────────────┐          ┌─────────(Start)─────────┐
│    Graph     │          ┌──────────────────────────────┐
│ Information  │─────────▶│  Shape Graph Information and   │───B1
└──────────────┘          │  Generate AI Input Information │
                          └──────────────────────────────┘
                                        │
                                        ▼
```

┌────────────────────────────────────────────────────┐
│                    Train Graph AI                    │───B2          103
│ Explanatory Variable:                                │
│   Variables Except for Cluster Name among            │            ┌──────────┐
│   Graph AI Input Information                          │──────────▶ │ Graph AI │
│ Response Variable:                                   │            └──────────┘
│   Cluster Name among Graph AI Input Information       │
└────────────────────────────────────────────────────┘
                        │
                        ▼

┌──────────────┐      ┌────────────────────────────────────────────────────┐
│ Amino-Acid   │      │              Infer Graph AI and                     │───B3
│  Statistic   │ ◀────│          Calculate Statistical Information          │◀──────┐
│ (e.g., Graph │      │ Explanatory Variable:                               │       │
│ Contribution)│      │   Variables Except for Cluster Name among Graph     │       │
└──────────────┘      │   AI Input Information                              │
                      └────────────────────────────────────────────────────┘
                                        │
                                        ▼
                                     ( End )

## INTERNATIONAL SEARCH REPORT

| International application No. |
|---|
| **PCT/JP2023/005471** |

| A. | CLASSIFICATION OF SUBJECT MATTER |
|---|---|

*G16B 40/00*(2019.01)i
FI:  G16B40/00

According to International Patent Classification (IPC) or to both national classification and IPC

| B. | FIELDS SEARCHED |
|---|---|

Minimum documentation searched (classification system followed by classification symbols)

G16B40/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2022/019331 A1 (TOHOKU UNIVERSITY) 27 January 2022 (2022-01-27) entire text, all drawings | 1-15 |
| A | US 2012/0265513 A1 (FANG, Jianwen et al.) 18 October 2012 (2012-10-18) entire text, all drawings | 1-15 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **13 April 2023** | **25 April 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/005471**

| Patent document cited in search report | | | Publication date (day/month/year) | Patent family member(s) | Publication date (day/month/year) |
|---|---|---|---|---|---|
| WO | 2022/019331 | A1 | 27 January 2022 | (Family: none) | |
| US | 2012/0265513 | A1 | 18 October 2012 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022019331 A
- JP 2022521686 A
- US 20120265513
- JP 2022527381 A
- US 20190266493